# EUROPEAN PATENT SPECIFICATION

(11) **EP 2 383 576 B1**
(45) Date of publication and mention of the grant of the patent: **03.04.2013**
(21) Application number: 11163935.7
(22) Date of filing: 27.04.2011
(51) Int. Cl.: G01N 33/543, B01L 3/00, H05K 3/00

(54) **Micro-electrode grid array for top and bottom recording from samples**
Mikroelektrodengitteranordnung zur oberen und unteren Aufzeichnung aus Proben
Réseau de grille de micro-électrodes pour l'enregistrement supérieur et inférieur à partir d'échantillons

(30) Priority: 28.04.2010 US 328847 P
(43) Date of publication of application: 02.11.2011
(73) Proprietor: IMEC, 3001 Leuven (BE); Katholieke Universiteit Leuven, K.U. Leuven R&D, 3000 Leuven (BE)
(72) Inventor: Eberle, Wolfgang, 3001 Leuven (BE); Braeken, Dries, 3900 Overpelt (BE); Huys, Roeland, 3012 Wilsele (BE); Prodanov, Dimiter, 1040 Etterbeek (BE)
(74) Representative: Hertoghe, Kris Angèle Louisa

(56) References cited:
- WO-A1-2005/098425
- GROSS ET AL: "Applications of microfluidics for neuronal studies", JOURNAL OF NEUROLOGICAL SCIENCES, ELSEVIER SCIENTIFIC PUBLISHING CO, AMSTERDAM, NL, vol. 252, no. 2, 8 January 2007 (2007-01-08), pages 135-143, XP005836716, ISSN: 0022-510X, DOI: DOI:10.1016/J.JNS.2006.11.009
- MORIN ET AL: "Investigating neuronal activity with planar microelectrode arrays: achievements and new perspectives", JOURNAL OF BIOSCIENCE AND BIOENGINEERING, ELSEVIER, AMSTERDAM, NL, vol. 100, no. 2, 1 August 2005 (2005-08-01), pages 131-143, XP005665578, ISSN: 1389-1723, DOI: DOI:10.1263/JBB.100.131
- HEUSCHKEL M O ET AL: "Buried microchannels in photopolymer for delivering of solutions to neurons in a network", SENSORS AND ACTUATORS B, ELSEVIER SEQUOIA S.A., LAUSANNE, CH, vol. 48, no. 1-3, 30 May 1998 (1998-05-30) , pages 356-361, XP004147365, ISSN: 0925-4005, DOI: DOI:10.1016/S0925-4005(98)00071-9
- BUCHER V ET AL: "ELECTRICAL PROPERTIES OF A LIGHT-ADDRESSABLE MICROELECTRODE CHIP WITH HIGH ELECTRODE DENSITY FOR EXTRACELLULAR STIMULATION AND RECORDING OF EXCITABLE CELLS", BIOSENSORS AND BIOELECTRONICS, ELSEVIER BV, NL, vol. 16, 1 January 2001 (2001-01-01), pages 205-210, XP001023717, ISSN: 0956-5663, DOI: DOI:10.1016/S0956-5663(01)00135-X

## Description

### Field of the invention

The present invention relates to the field of interface devices for providing an access to the electrical activity of a network of cells through a set or matrix of a plurality of elements, said elements comprising micro-fluidics and electrical and/or optical multi-electrode arrays.

Furthermore the present invention relates to a device suitable for observing and recording long term neuronal processes.

### Background of the invention

In many cell-physiological and medical applications, there is a demand to measure biochemical and physiological parameters in the close vicinity of living cells. A popular technique for recording of network neural activity in vitro is the Multi-Electrode Array (MEA) (see for example WO 2005/098425). A MEA is a device that contains multiple shanks through which neural signals are obtained or delivered, essentially serving a neural interfaces that connect neurons to electronic circuitry. MEAs are made of an insulating substrate (e.g. glass), with a maximum of around 100 electrodes and a minimal electrode pitch of around 10 µm. Freshly prepared tissue slices can be applied or cultivated on the MEA, for signal recordation and/or for stimulation.

Certain in-vitro physiological experiments require more improved functionalities of the multi-electrode arrays (MEAs), for example studies of long-term potentiation and depression. For this type of application, the MEAs that are commercially available are poorly suited, for one or more of the following reasons:
1. Perfusion in the bottom part of the slice is either suboptimal leading to hypoxia and subsequent death of the tissue cells, or in systems featuring integrated fluidics, bottom-recording may be disturbed by the perfusion pattern which is also situated at the bottom. Integrating the perfusion and the electronics in one chip makes also the system expensive.
2. Due to limited space for wires, current systems play a trade-off between interelectrode distance and the area coverage of the structures of interest. Thus, the spatial resolution on the recorded area is limited, and for a lot of applications not sufficient.
3. Due to a bad seal between the tissue slice and the recording electrodes in the MEA, the recordings are often contaminated with noise.

Hence, in view of the above disadvantages, there is a definite need for an array-like interface system that allows high-density simultaneous long-term recordings from tissue slices, e.g. hippocampal slices, in vitro.

### Summary of the invention

It is a goal of the present invention to solve the problems related to existing MEA's for recording and/or stimulating.

It is an object of embodiments of the presents invention to provide a good interface device for providing access to a tissue slice such as for example a brain slice or a cardiac slice, or a cell culture, whereby the interface device is arranged for culturing, stimulating and/or detecting the neuronal behavior of said tissue slice or cell culture. Such interface device is an important tool in the field of neurophysiology.

The above objective is accomplished by a device and a method according to embodiments of the present invention.

In a first aspect, the present invention provides a mixed micro-fluidic multi-electrode grid array device suitable for holding a tissue slice, e.g. a brain slice or a cardiac slice, or a cell culture and for recording and/or stimulating neuron cells from said tissue slice or cell culture. The device comprises:
a first bottom substrate in the form of a stack made of a grid comprising an electrical and/or optical multi-electrode array and a backbone underneath said grid comprising a micro-fluidic perfusion system,
a second top substrate in the form of a grid comprising at least an electrical and/or optical multi-electrode array,
means for pressing and positioning said first and second substrate together and adhering a tissue slice or a cell culture in between said two substrates.

It is an advantage of a mixed micro-fluidic multi-electrode grid array device according to embodiments of the present invention that it can function as an interface device which provides a multi-electrode array-system (MEA) that allows for making high-density simultaneous long-term recordings and/or stimulations from tissue slices, e.g. brain (hippocampal) slices or cardiac slices, or cell cultures in-vitro. A grid array device according to embodiments of the present invention may further achieve one or more of:
● An improved functionality of the MEAs;
● An improved spatial resolution for the recorded area;
● An improved seal between the tissue slice or cell culture and the recording electrodes to avoid contamination.

The problems related to existing MEA systems are solved by the advanced MEA system of embodiments of the present invention which is further referred to as a mixed system micro-fluidic multi-electrode grid array (MEGA). The problems are solved by combining an improved micro-fluidic perfusion system with an improved electrical and/or optical multi-electrode grid array. The MEGA system according to embodiments of the present invention is further differentiated from existing MEA systems by separating the micro-fluidics and the electrode grid from each other into two separatable structures, which allows a separate development of the electrode array (optionally including electronic circuitry such as a chip-part) and the micro-fluidics part. Furthermore fabrication of a grid without micro-fluidics gives more freedom in suitable grid heights (thicknesses).

It is an advantage of the MEGA system according to embodiments of the present invention that the thickness of at least one of the grids may be reduced after processing by e.g. grinding. The thickness of the grid may be adapted which gives an increase in the flexibility of e.g. the top grid to allow following the (brain) tissue slice shape. This has the advantage that the thickness of a grid without micro-fluidics can be chosen or adapted such that its thickness is suitable for adhering closely to the brain tissue when pressing both top and bottom substrates together.

The MEGA system according to embodiments of the present invention is further differentiated from existing MEA systems used for recording neuron cells in tissue slices by providing at least a top substrate (top grid) and a bottom substrate (bottom grid) and wherein the tissue slice is sandwiched in between said both substrates (grids). By providing said at least two substrates it is possible to provide a micro-fluidic perfusion system on one substrate and to provide an electrode grid system on the other substrate (or alternatively a combination of both systems on the two substrates), thereby creating more spatial resolution for the recorded area and more flexibility towards functionality of the electrodes. The tissue slice of interest is placed in between both substrates and by pressing both substrates tightly an improved sealing may be achieved thereby avoiding contamination.

It is an advantage of the MEGA system according to embodiments of the present invention that the micro-fluidic system may be situated on a separate substrate such that a more flexible micro-fluidic system can be used being able to provide the tissue slice with e.g. oxygen, growth factors, and other (bio) chemical support. Therefore the bottom substrate is preferably made of a stacking of a bottom layer made of a polymeric material and comprising the micro-fluidic system (also referred to as the backbone) with the multi-electrode grid array on top of or fit in a cavity of said backbone having a suitable shape, e.g. a shape adapted for receiving the multi-electrode grid array.

It is an advantage of the MEGA system according to embodiments of the present invention that electrodes may be provided on the at least one of bottom and/or top grids in a single-layer configuration or (preferred for higher electrode density) in a multi-layer wiring configuration thereby forming an interconnect frame around the grid.

It is an advantage of the MEGA system according to embodiments of the present invention that the grid can be manufactured in CMOS-compatible way whereby the same manufacturing flow can be used for manufacturing the bottom and top grid. Additional (optional) steps may be added e.g. to modify the thickness of the grid for top or bottom use. The bottom grid may be used with electrodes on top and it is preferably flipped when used as a top grid (with electrodes facing downwards towards the tissue slice or the cell culture). A same interconnect mechanism can be used for both grids to connect the electrodes electrically to external electronics or to electronics embedded in the grid frame. It is a further advantage that by providing a multi-electrode grid array on both the top and bottom surface of a tissue slice, e.g. brain slice, or cell culture it becomes possible to stimulate and record the behavior of a single neuron cell of a tissue slice, e.g. brain slice, or cell culture simultaneously.

It is an advantage of the MEGA system according to the present invention that further electronic circuitry, e.g. CMOS-based electronics, nano-electronics, GaN and other III-IV based technology, post-CMOS electronics and/or bio-electronics, can be added to the MEGA system. It is an advantage that by providing a GaN comprising substrate and/or component to the MEGA system according to embodiments of the present invention, optical stimulation of the neuron cells can be easily achieved.

It is a further advantage of the MEGA system according to embodiments of the present invention that the thickness of at least one of the grids may be reduced after processing by e.g. grinding. The height of e.g. the bottom grid may be reduced such that only shallow grooves in the grid comprising micro-fluidics are required and the grid is not sticking out significantly and further allows an unconstrained neuronal growth across this entire grid.

It is a further advantage of the MEGA system according to embodiments of the present invention that the thickness of at least one of the grids may be adapted (reduced) on purpose such that a height difference may be created between the bottom grid (preferably fit in a backbone comprising the micro-fluidics) and the top grid. Said height difference may be used to confine areas for e.g. perfusion or micro-fluidic release, protect areas from each other (to avoid micro-fluidic disturbance), to confine neuronal growth,....

It is an advantage of the MEGA system according to embodiments of the present invention that electronics can be embedded in the grid frame or in the electronic grid itself such that direct embedding of CMOS electronics close to the electrodes can be achieved.

It is a further advantage of the MEGA system according to embodiments of the present invention that the grid can use a single-layer interconnect system or a multi-layer interconnect thereby using standard available CMOS interconnect processes.

It is a yet further advantage of the MEGA system according to embodiments of the present invention that electrodes can be provided in top and bottom grid, whereby said electrodes can be aligned or misaligned with respect to one another on purpose. Alignment structures can be used to align both grids perfectly on each other. This allows electrode-on-top-of-electrode stimulation or recordings from the very same position.

The MEGA system according to embodiments of the invention may be further completed by incorporating the whole MEGA system in a closed chamber. Said chamber may be made of a transparent material such that the MEGA system can be seen and inspected during operation. The advantage of incorporating the MEGA system in a closed system is that a controlled environment can be created which is optimized e.g. for keeping the brain slices alive and e.g. to avoid contamination.

The MEGA system according to embodiments of the invention may be further completed by adding intermediate grid structures such that e.g. multiple tissue slices, e.g. brain slices, or cell cultures can be recorded and or stimulated at the same time in one MEGA system. This has the additional advantage that interaction between different brain slices can be monitored.

It is an advantage of a mixed micro-fluidic multi-electrode grid array device according to embodiments of the present invention that it may provide an interface device for providing access to both the top and bottom surface of a tissue slice or cell culture, such that it becomes possible to simultaneously stimulate and record the behavior of a single neuron cell of a tissue slice.

It is an advantage of a mixed micro-fluidic multi-electrode grid array device according to embodiments of the present invention that it allows running experiments on thousands of neurons simultaneously and studying their interaction and behavior not only from the bottom side but also from the top side of the tissue slice or cell culture.

It is an advantage of a mixed micro-fluidic multi-electrode grid array device according to embodiments of the present invention that it allows to provide access to a (living) tissue slice or cell culture whereby it becomes possible to separate the required micro-fluidic perfusion system from the required electrical (optical) multi-electrode grid array used to stimulate and/or record the neuron cells of a tissue slice or a cell culture.

The MEGA system according to embodiments of the invention may be used to study neuronal processes or aspects of muscle physiology such as spreading of excitation in a network of cells and/or neuronal processes such as long-term potentiation (LTP) and long-term depression (LTD) in the hippocampus, an important brain region for spatial and associative memories.

In a second aspect, the present invention provides a method for manufacturing a mixed micro-fluidic multi-electrode grid array device. The method comprises:
obtaining a first and second supporting substrate,
providing a grid structure in said first and second supporting substrate,
providing an electrical and/or optical multi-electrode grid array onto said first and second supporting substrate, and
providing a backbone made of a polymeric material underneath said second substrate and providing a micro-fluidic perfusion system in said backbone.

A method according to embodiments of the present invention may furthermore comprise providing, electrically connected to said multi-electrode grid array, at least one of a chip, a biosensor, an optical sensor, an optical stimulator.

A method according to embodiments of the present invention may furthermore comprise providing means for positioning and pressing the first and second supporting substrates together and holding a living tissue slice or cell culture in between the two substrates.

A method according to embodiments of the present invention may furthermore comprise adding at least one intermediate substrate situated in between said first and second supporting substrate, the at least one intermediate substrate being in the form of a grid comprising at least an electrical and/or optical multi-electrode array and optional micro-fluidics.

Particular and preferred aspects of the invention are set out in the accompanying independent and dependent claims. Features from the dependent claims may be combined with features of the independent claims and with features of other dependent claims as appropriate and not merely as explicitly set out in the claims.

These and other aspects of the invention will be apparent from and elucidated with reference to the embodiment(s) described hereinafter.

### Brief description of the drawings

FIG. 1 illustrates a schematic representation of a MEGA device according to an embodiment of the present invention.
FIG. 2 illustrates one embodiment of a grid for use in a MEGA device according to embodiments of the present invention.
FIG. 3 illustrates one embodiments of a backbone for use in a MEGA device according to embodiments of the present invention, which backbone fits with the grid illustrated in FIG. 2.
FIG. 4 illustrates introduction of a grid as in FIG. 2 into a backbone as in FIG. 3.
FIG.5 5 schematically illustrates a MEGA device according to embodiments of the present invention, comprising a stack having three grids, a tissue slice being placed between any two adjacent grids, and a backbone being provided underneath one of the grids, the backbone comprising a microfluidics part.
FIG. 6 illustrates another embodiment of a grid for use in a MEGA device according to embodiments of the present invention.
FIG. 7 illustrates the grid of FIG. 6 introduced into a corresponding backbone.

The drawings are only schematic and are non-limiting. In the drawings, the size of some of the elements may be exaggerated and not drawn on scale for illustrative purposes.

Any reference signs in the claims shall not be construed as limiting the scope.

In the different drawings, the same reference signs refer to the same or analogous elements.

### Detailed description of illustrative embodiments

As used herein and unless stated otherwise, the term "MEA" relates to a multi electrode Array system provided onto an insulating substrate (e.g. glass). The array of electrodes may typically have a maximum of around 100 electrodes having a diameter in the range of 10-50 µm and a minimal electrode pitch of around 25-50µm.

As used herein and unless stated otherwise, the term "tissue slice" refers to sections or explants of tissue which are maintained in culture. Particular examples of tissue slices in the context of the present invention are brain slices and cardiac slices, the present invention, however, not being limited thereto. As an example, brain slice cultures can be derived from sections of the whole brain tissue or from explants obtained from specific regions of the brain. Any region can be used to generate a brain slice. However, a preferred source of a brain slice is explants obtained from specific regions of the brain, for example the hippocampus region.

As used herein and unless stated otherwise, the term "cell culture" relates to the complex process by which cells are grown under controlled conditions and cells thus grown. Cells are grown and maintained at appropriate culture conditions (e.g. temperature, gas mixture, growth medium; culture conditions may vary widely for different cell types).

As used herein and unless stated otherwise, the term "micro-fluidic system" relates to a network of channels, for example made out of polyethylene glycol (PEG) or polydimethylsiloxane (PDMS), for providing a tissue slice, for example a brain slice or a cardiac slice, or a cell culture with the appropriate culture conditions, including oxygen, growth factors, and other (bio) chemical support to keep the tissue slice or cell culture alive. Specific examples of suitable microfluidic systems in the context of the present invention, as well as methods of their manufacturing, are described by T. Stieglitz in "Integration of Microfluidic Capabilities into Micromachined Neural Implants", International Journal of Micro-Nano Scale Transport, Vol.1, N°2, 2010.

As used herein and unless stated otherwise, a "growth factor" refers to a naturally occurring substance capable of stimulating cellular growth, proliferation and cellular differentiation. It may typically be a protein or a steroid hormone, the present invention nevertheless not being limited thereto.

As used herein and unless stated otherwise, a "biocompatible material" refers to a material that is compatible to cells or tissues, without adversely affecting cellular physiology.

The invention will now be described by a detailed description of a device according to a first aspect of the invention and a method for fabricating said device according to a second aspect of the invention. Although several embodiments of the invention are described by way of example, it must be clear that other embodiments of the invention can be configured according to the knowledge of persons skilled in the art without departing from the technical teaching of the invention, the invention being limited only by the terms of the appended claims.

According to a first aspect of the invention a mixed system micro-fluidic multi-electrode grid array (MEGA) is disclosed. Said MEGA system is a device which is suitable for top or bottom surface recording/stimulation on tissue (e.g. brain or cardiac) slices or cell cultures and which is alternatively suitable for simultaneous top and bottom surface recording/stimulation on tissue slices or cell cultures. Hereto, the tissue slice or cell culture is held between a top and a bottom substrate comprising at least an electrical and/or optical multi-electrode array. The MEGA system furthermore comprises a microfluidic perfusion system for providing the tissue slice or cell culture with appropriate culture conditions for keeping the tissue slice or cell culture alive. The tissue slice or cell culture typically has a thickness between 250-400 um.

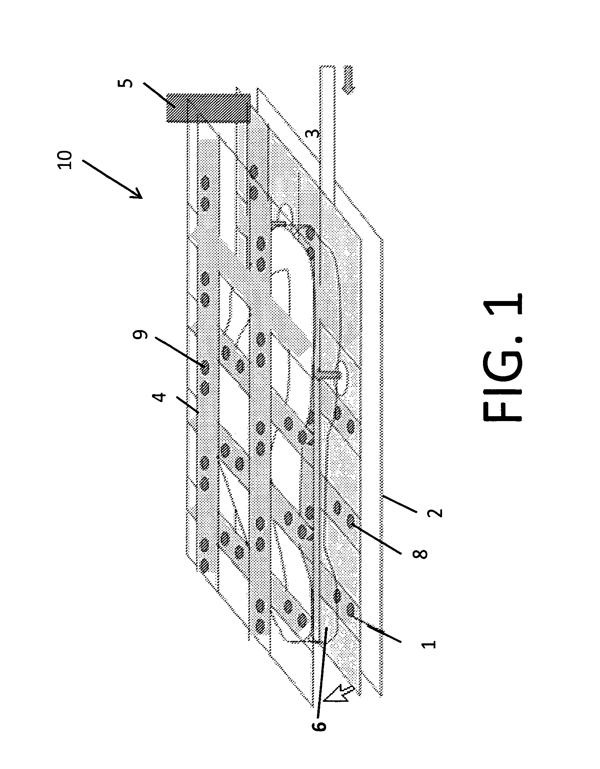

FIG. 1 illustrates a schematic representation of a MEGA system 10 according to an embodiment of the present invention. The MEGA system 10 as shown in the example comprises a bottom surface in the form of a grid 1. This grid 1 comprises at least an electrical and/or optical multi-electrode array. The multi-electrode array comprises an array of electrodes, which in the example illustrated are indicated by the black dots 8. The MEGA system 10 furthermore comprises a top surface in the form of a second grid 4. Also this grid 4 comprises at least an electrical and/or optical multi-electrode array. The multi-electrode array comprises an array of electrodes, which in the example illustrated are indicated by the black dots 9. The grids 1, 4 may furthermore be provided with electronic components, e.g. a chip comprising such electronic components, electrically connected to the electrodes 8, 9. Furthermore, a backbone 2 is provided underneath said first grid 1. The backbone 2 provides mechanical support and strength to the system 10. The backbone 2 is furthermore provided with a micro-fluidic system 3. The micro-fluidic system 3 may be made of biocompatible polymer. The grids 1, 4 may be kept in position with the help of means 5 for positioning the bottom surface and the top surface with respect to one another, and for adhering a tissue slice 6 or cell culture in between both substrates. Such means 5 may for example be a microdrive to adapt the vertical direction of any of the grids 1, 4.

The MEGA device according to embodiments the invention is suitable for holding a tissue slice, e.g. a brain slice 6, or a cell culture and for recording and/or stimulating neuron cells from said tissue slice or cell culture.

The grid 1, 4 of the first substrate and/or the second substrate of the MEGA device 10 according to embodiments of the invention may be made of a suitable insulating material, such as e.g. Silicon, glass, Silicon on insulator (SOI), GaN, Polymers. The grid may preferably be made of biocompatible material. On the grid 1, 4 an electrical and/or optical array comprising a plurality of electrodes 8, 9 is provided. The electrodes 8, 9 within one array may be individually addressable. Hereto, addressing circuitry may be provided on the grid 1, 4. Furthermore, a grid 1, 4 of the MEGA device 10 according to embodiments of the invention may comprise electronic circuitry electrically connected to the plurality of electrodes 8, 9, for example at least one of a chip, a biosensor, an optical sensor, an optical stimulator, an electrical sensor, or an electrical stimulator.

In particular embodiments of the present invention, a grid 1, 4 of the MEGA device 10 may comprise electrodes in a single-layer configuration. Alternatively, the electrodes 8, 9 may be laid out in a multi-layer wiring configuration thereby forming an interconnect frame (not illustrated in the drawings) around the grid 1, 4.

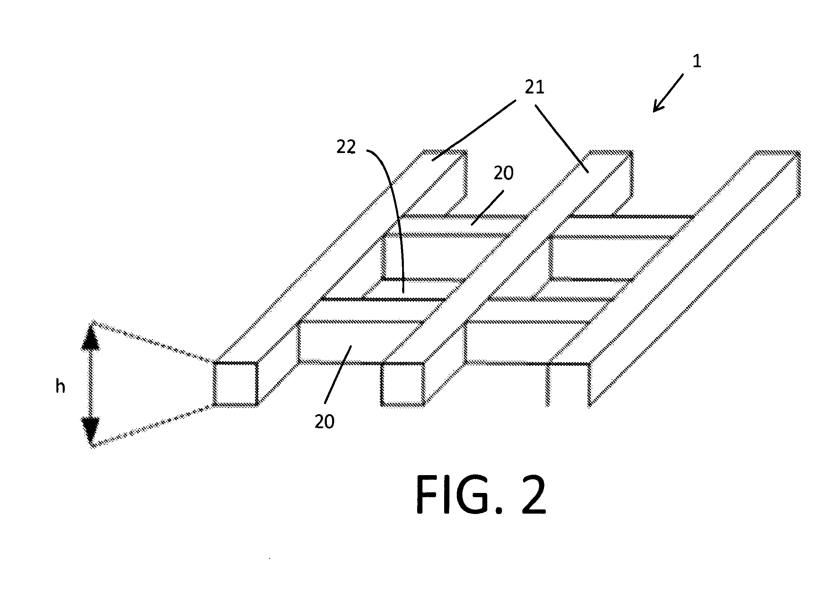

An example of a grid 1 according to embodiments of the present invention is illustrated in FIG. 2. Such grid 1 consists of a network of bars in at least two different directions. In the embodiment illustrated in FIG. 2, the grid 1 comprises first bars 20 in a first direction, e.g. horizontal direction, and second bars 21 in a second direction, which may be, but does not need to be perpendicular to the first direction, e.g. vertical direction. The bars 20, 21 may be evenly spaced. In embodiments of the present invention, the spacing between all first bars 20 may be equal, and the spacing between all second bars 21 may be equal. The spacing between the first bars 20 may be, but does not need to be, equal to the spacing between the second bars 21. The grid provides a support for the plurality of electrodes forming a multi-electrode array. FIG. 2 illustrates an example of a grid 1, but also other grids, such as e.g. a top grid or an intermediate grid as explained below, according to embodiments of the present invention may have a same or similar lay-out.

The grid 1, 4 of the MEGA device 10 according to embodiments of the invention has grid fields 22, 23, e.g. the spaces delimited by two first bars 20 in the first direction and by two second bars 21 in a second direction. Grid fields at an edge of the grid 1, 4 may be, but do not need to be, delimited by two first bars 20 and only one second bar 21, as illustrated in FIG. 2, or by only one first bar 20 and two second bars 21 (not illustrated), or, at a corner, even by only one first bar 20 and only one second bar 21 (not illustrated). The grid fields 22, 23 may have dimensions in the order of 10-50 µm. The array of electrodes on said grid may typically have a maximum of around 100 electrodes, each having a diameter in the range of 10-50 µm and a minimal electrode pitch of around 25-50µm.

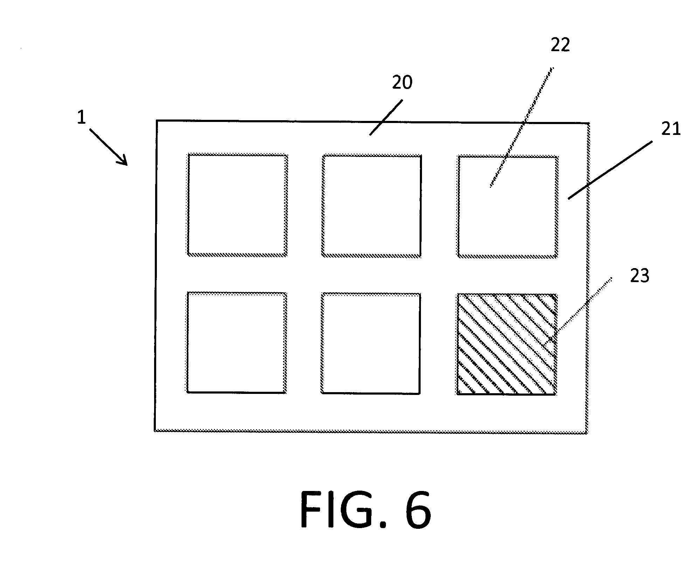

Another example of a grid 1 according to embodiments of the present invention is illustrated in FIG. 6. Also in this embodiment the grid comprises first bars 20 in a first direction and second bars 21 in a second direction. In the embodiment illustrated, the first direction and the second direction are perpendicular to one another, but the invention is not limited thereto. In the embodiment illustrated, the bars are evenly spaced, and this in both directions; the present invention, however, is not limited thereto.

In embodiments of the present invention, the grid fields may either be empty grid fields 22 (grid openings), or functional grid fields 23. Empty grid fields 22 are illustrated in FIG. 6 as white squares. Such empty grid fields are just openings, for example obtained by etch through. This means they are transparent, and cells and tissue can grow through these empty grid fields 22. A functional grid field 23 is illustrated in FIG. 6 by means of a hatched square. A functional grid field 23 can contain functional features. The functional features may be, but do not need to be, the same as the features on the grid itself, e.g. electrodes and/or CMOS features such as electronic circuitry. It is advantageous is a substantial amount of grid fields, e.g. more than 75%, more particularly more than 90%, for example more than 95%, remain empty to facilitate growth of tissue or cells through these empty grid fields 22. Functional fields 23 may be placed where a higher density of electrodes is desired, e.g. for matching the biological topology of a tissue slice or cell culture.

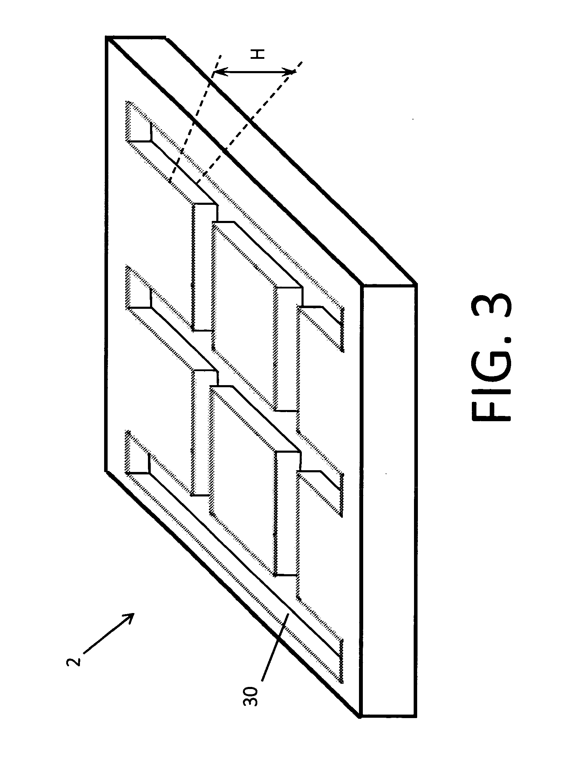

In accordance with embodiments of the present invention, at least the first grid 1 may be supported by a backbone 2. The backbone 2 is adapted with grooves 30 adapted for receiving the grid 1. Hence the grooves 30 at least correspond to the first bars 20 and the second bars 21. A backbone 2 adapted for co-operation with the grid 1 as illustrated in FIG. 2 is illustrated in FIG. 3. Optionally, not illustrated in the drawings, there may be more grooves or grooves at other locations than where the first bars 20 and second bars 21 are to be received. This allows to have a single backbone 2 for a plurality of types of grids. The backbone 2 furthermore comprises channels and optionally chambers for providing a microfluidic system, more particularly e.g. a microfluidic perfusion system. The backbone 2 may be made of any suitable material, such as polyethylene glycol (PEG) or polydimethylsiloxane (PDMS). The microfluidic system in the backbone 2 is adapted for providing the tissue slice 6 or cell culture with appropriate culture conditions. In particular, the microfluidic system may be adapted for providing the tissue slice 6 or cell culture with oxygen, growth factors, and/or other (bio) chemical support to keep the tissue slice 6 or cell culture alive. It is an advantage of embodiments of the present invention that the addition of a micro-fluidic perfusion system such as micro-fluidic channels make it possible to provide supporting medium to the living tissue/cells.

The grid 1 has a grid height h, as illustrated in FIG. 2. The height h of the grid 1 is determined by the thickness of the first bars 20 and the second bars 21. The grooves 30 in the backbone 2 have a depth H. The depth H may be such that the grid 1 fits in height direction into the backbone 2. It is advantageous if the depth H of the grooves 30 in the backbone 2 is not larger than the height h of the grid 1, in order to have all electrodes lying in a plane.

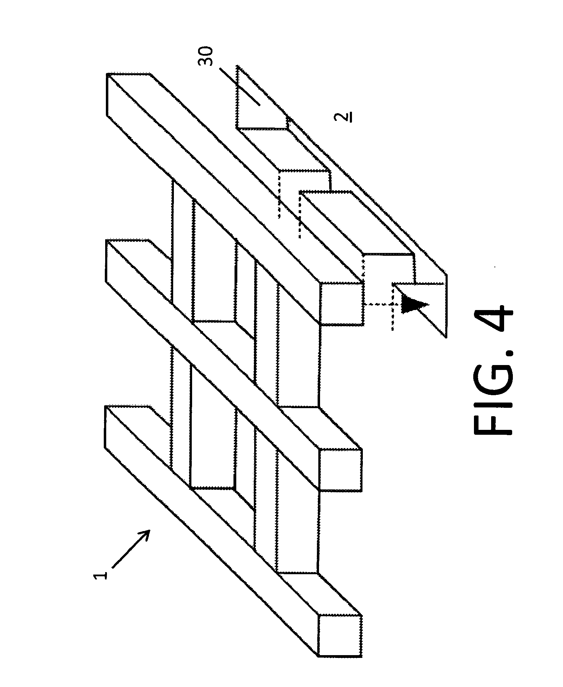

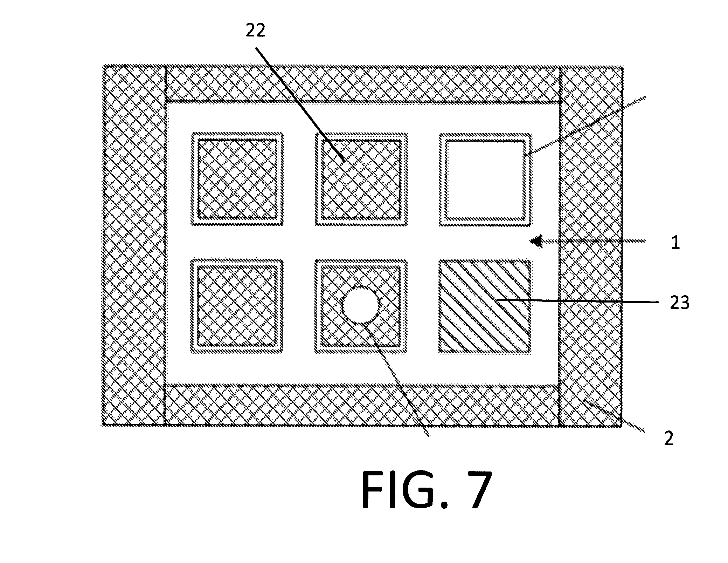

A first substrate may be made by fitting a first grid 1 into a backbone 2, as illustrated in FIG.4 and FIG.7 for the two grid embodiments as illustrated in FIG.2 and FIG. 6, respectively. Such first substrate can be used as such for supporting, stimulating and/or sensing a tissue slice and/or a cell culture. This structure is advantageous per se, as it may provide a combination of an electronic interface (the multi-electrode array on the grid), which can advantageously be made of or on a first material such as a semiconductor material, e.g. silicon, with a microfluidic component, which can advantageously be made of a second material, e.g. PDMS. PDMS is advantageous for forming microfluidics, but does not allow easy manufacturing of electronics. Equally, semiconductor material is good for making electronic components, but is less suited for manufacturing microfluidics components. A first substrate as in FIG. 4 combines both advantages, thus at the same time alleviating the disadvantages. Such system comprising a first substrate comprising a first grid 1 into a backbone 2, the first grid 1 comprising a high density recording and stimulating electrode matrix, can be used to simultaneously stimulate (either electrically or optically) and record tissue or cell culture response.

From the embodiment illustrated in FIG. 7 it can be seen that the empty fields 22 are transparent: parts of the backbone 2 can be seen through the empty fields 22. These parts may include microfluidic functionality. Some of the empty fields may be filled with a substrate, or may be left empty to be filled e.g. with a microfluidics PDMS mold.

The backbone 2 and/or the grid 1 of the MEGA device according to embodiments of the invention may further comprise electronic circuitry, such as e.g. CMOS-based electronics, nano-electronics, GaN and other III-IV based technology electronics, post-CMOS electronics and/or bio-electronics.

In use, for forming a MEGA device 10 according to embodiments of the present invention (as illustrated in FIG. 1), a first substrate is formed by fitting a grid 1 into the grooves 30 of a backbone 2, as illustrated in FIG. 4. On top of the first substrate, a tissue slice 6 or a cell culture is applied. On top thereof, a second grid 4 is applied, which second grid 4 may physically look like first grid 1. This second grid 4 is provided with at least an electrical and/or optical electrode array comprising a plurality of electrodes 9 (multi-electrode array). The second grid 4 may be flipped around such that its electrodes 9 face the tissue slice 6 or cell culture. If both the electrodes 8 on the first grid 1 and the electrodes 9 on the second grid 4 are brought into contact with the tissue slice 6 or cell culture, simultaneous measurements can be performed at the bottom (in contact with the first grid 1) and at the top (in contact with the second grid 4) of the tissue slice 6 or cell culture. This way, a 3D resolution may be obtained, i.e. measurements may be performed at different levels of the tissue slice 6 or cell culture. Alternatively, stimulation may be performed at the bottom side, while measurements may be performed at the top side, or vice versa. Yet alternatively, the bottom and the top of the tissue slice 6 or cell culture can be stimulated simultaneously, while measurements can also be performed at both the top and the bottom side of the tissue slice 6 or cell culture.

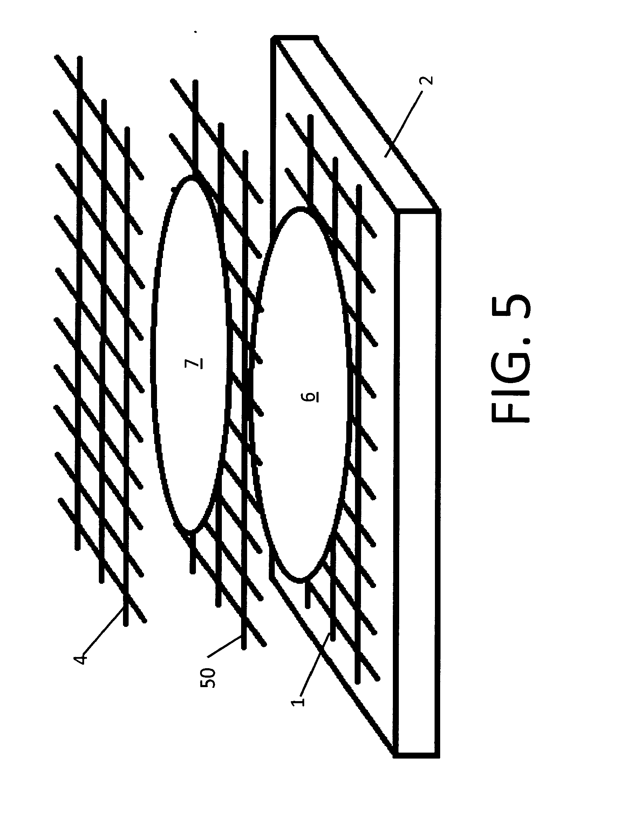

In alternative embodiments, a MEGA device 10 can not only comprise a first, bottom, substrate comprising a backbone 2 and a grid 1, and a second, top, substrate comprising a grid 4. A MEGA device 10 could furthermore comprise at least one intermediate substrate comprising a grid 50. This way, a stack of tissue slices 6, 7 and/or cell cultures can be made, with grids 50 comprising at least an electrical and/or optical multi-electrode array in between. An example is schematically illustrated in FIG. 5. Electrodes of the multi-electrode array may be applied on the intermediate grids 50 either at a single side thereof, or at both sides. If electrodes are only present at one side of the grid 50, the intermediate grid 50 can be flipped or non-flipped, depending on the desired location of the electrodes. It is an advantage of this embodiment that by adding intermediate grids 50 more than one tissue slice 6, 7 and/or cell culture can be studied simultaneously. Optionally, the interaction between the different tissue slices 6, 7 and/or cell cultures can be monitored. Optionally (not illustrated in the drawings), any or each of the intermediate grids 50 can be provided with suitable microfluidics, for example a backbone as backbone 2.

The stacking of grids 1, 4, 50 in accordance with embodiments of the present invention provides possibilities of either (i) simultaneous readouts from several tissue slices or cell cultures, or (ii) can be used to prove possibilities for tissue engineering and integration in the context of long-term culturing of slices. In addition, temperature sensors may be integrated to provide real-time readout. Such read-out can improve the control over the experiment and provide information to perform continuous quality control of the experimental conditions.

The MEGA device 10 according to embodiments of the invention may be placed in a closed chamber (not illustrated in the drawings). Said closed chamber may be made of a transparent material to inspect MEGA system 10 during operation. The closed chamber may be adapted to provide a controlled environment which may be further optimized e.g. for keeping the tissue slices 6, 7 and/or cell cultures alive and/or e.g. to avoid contamination.

The MEGA device 10 according to embodiments of the invention may be used for stimulating and/or recording neuron or muscle cells of a living tissue.

The MEGA device 10 can be used to perform fine-grained examination of the electrical activity of the excitable cells (for example, the spread of changes in neuronal plasticity) in tissue slices, e.g. brain slices or cardiac slices, in vitro. The implementation of a micro-perfusion system, as part of the microfluidic system, on the electrodes would enable long-term recordings in acute slices, perfusion isolation of certain areas of the slice and their differential pharmacological manipulation (for example characterization of the activated compared non-activated areas) and other similar pharmacological applications. The fluid and chemical fluxes of the micro-fluidic part can be further simulated in order to provide minimal disturbance of the bottom-part electrical and/or optical recording.

The MEGA device 10 according to embodiments of the invention may be used to study aspects of muscle physiology, such as spreading of excitation in a network of cells, and neuronal processes, such as the long-term potentiation (LTP) and long-term depression (LTD) in the brain.

According to a second aspect of the invention a method for manufacturing a mixed micro-fluidic multi-electrode grid array device (MEGA device) 10 according to embodiments of the invention (as described above) is disclosed. Said method comprises at least steps of:
- obtaining a first and second supporting substrate,
- providing a first and second grid 1, 4 in said first and second supporting substrate, respectively,
- Providing an electrical and/or optical multi-electrode grid array onto said first and second grid 1, 4, and
- providing a backbone 2, for example made of a polymeric material, with a shape adapted for a close fit with the first grid 1 and providing a micro-fluidic perfusion system in said backbone 2.

It is an advantage of the present invention that conventional processing steps can be used for manufacturing the different components of the MEGA device 10 according to embodiments of the present invention.

The method for manufacturing the MEGA device 10 according to embodiments of the invention may further comprise the step of providing at least one of a chip, a biosensor, an optical sensor, an optical stimulator, a biochemical sensor, a biochemical stimulator electrically coupled to the multi-electrode grid array on said first and/or second grid 1, 4.

The method for manufacturing MEGA device according to embodiments of the invention may furthermore comprise the step of providing means 5 for pressing the grids 1, 4 together and holding a living tissue such as a tissue slice e.g. a brain slice or a cardiac slice, or a cell culture there between.

The method for manufacturing MEGA device 10 according to embodiments of the invention may furthermore comprise the step of adding intermediate grids 50 situated in between said first grid 1 and second grid 4 and also in the form of a grid comprising at least an electrical and/or optical multi-electrode array and optional micro-fluidics.

Other variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

The foregoing description details certain embodiments of the invention. It will be appreciated, however, that no matter how detailed the foregoing appears in text, the invention may be practiced in many ways, and is therefore not limited to the embodiments disclosed. It should be noted that the use of particular terminology when describing certain features or aspects of the invention should not be taken to imply that the terminology is being re-defined herein to be restricted to include any specific characteristics of the features or aspects of the invention with which that terminology is associated.

## Claims

1. A mixed micro-fluidic multi-electrode grid array device (10) suitable for holding a tissue slice (6) or a cell culture and for recording and/or stimulating neuron cells from said tissue slice (6) or cell culture, the device (10) comprising:
a first bottom substrate in the form of a stack made of a grid (1) comprising an
electrical and/or optical multi-electrode (8) array and a backbone (2) underneath said grid (1) comprising a micro-fluidic perfusion system,
a second top substrate in the form of a grid (4) comprising at least an electrical and/or
optical multi-electrode (9) array,
means (5) for pressing and positioning said first and second substrate together and
adhering a tissue slice (6) or a cell culture in between said two substrates.

2. A device (10) according to claim 1, wherein the first substrate and/or the second substrate comprise Silicon, glass, Silicon on insulator (SOI), GaN, Polymers.

3. A device (10) according to any of the foregoing claims, wherein any of said grids (1, 4) comprising at least an electrical and/or optical multi-electrode array furthermore comprises at least one of electronic circuitry, a chip, a biosensor, an optical sensor, an optical stimulator, an electrical sensor, an electrical simulator.

4. A device (10) according to any of the foregoing claims, wherein any of said grids (1, 4) comprising at least an electrical and/or optical multi-electrode array comprises electrodes in a single-layer configuration or alternatively in a multi-layer wiring configuration thereby forming an interconnect frame around the grid.

5. A device (10) according to any of the foregoing claims, wherein the micro-fluidic perfusion system comprises a network of channels made out of polyethylene glycol (PEG) or polydimethylsiloxane (PDMS), for providing the tissue slice (6) or cell culture with oxygen, growth factors, and/or other (bio) chemical support to keep the tissue slice (6) or cell culture alive.

6. A device (10) according to any of the foregoing claims, wherein the first substrate and/or the second substrate further comprise any of CMOS-based electronics, nano-electronics, GaN and other III-IV based technology, post-CMOS electronics and/or bio-electronics.

7. A device (10) according to any of the foregoing claims, wherein the first substrate and/or the second substrate in the form of a grid has openings (22) in the order of 10-50 µm.

8. A device (10) according to any of the foregoing claims, wherein the device (10) is placed in a closed chamber.

9. A device (10) according to any of the foregoing claims, further comprising intermediate substrates situated in between said first and second substrate and in the form of a grid (50) comprising at least an electrical and/or optical multi-electrode array and optional micro-fluidics.

10. Use of A device (10) according to any of the foregoing claims for stimulating and/or recording neuron or muscle cells of a living tissue.

11. Use according to claim 10 for studying aspects of muscle physiology, and/or neuronal processes.

12. A method for manufacturing a mixed micro-fluidic multi-electrode grid array device (10), the method comprising:
obtaining a first and second supporting substrate,
providing a grid structure in said first and second supporting substrate,
providing an electrical and/or optical multi-electrode grid array onto said first
and second supporting substrate,
providing means for positioning and pressing the first and second supporting
substrates together and holding a living tissue slice or cell culture in between the two substrates, and
providing a backbone made of a polymeric material underneath said second
substrate and providing a micro-fluidic perfusion system in said backbone.

13. A method according to claim 12, furthermore comprising providing, electrically connected to said multi-electrode grid array, at least one of a chip, a biosensor, an optical sensor, an optical stimulator.

14. A method according to any of claims 12 or 13, furthermore comprising adding at least one intermediate substrate situated in between said first and second supporting substrate, the at least one intermediate substrate being in the form of a grid comprising at least an electrical and/or optical multi-electrode array and optional micro-fluidics.

## Patentansprüche

1. Gemischte mikrofluidische Multielektrodengitteranordnungsvorrichtung (10) zum Halten eines Gewebeschnittes (6) oder einer Zellkultur und zur Aufzeichnung und/oder Stimulierung von Nervenzellen aus dem Gewebeschnitt (6) oder der Zellkultur, wobei die Vorrichtung (10) Folgendes umfasst:
ein erstes unteres Substrat in Form eines Stapels aus einem Gitter (1), das eine elektrische und/oder optische Multielektrodenanordnung (8) umfasst, und
einem Gerüst (2) unter dem Gitter (1), das ein mikrofluidisches Perfusionssystem umfasst,
ein zweites oberes Substrat in Form eines Gitters (4),
das zumindest eine elektrische und/oder optische Multielektrodenanordnung (9) umfasst,
Mittel (5) zum Zusammendrücken und Positionieren der ersten und zweiten Substrate und zur anhaftenden Befestigung eines Gewebeschnitts (6) oder einer Zellkultur zwischen den beiden Substraten.

2. Vorrichtung (10) nach Anspruch 1, worin das erste Substrat und/oder das zweite Substrat Silikon, Glas, Silikon-auf-Isolator (SOI), GaN, Polymere umfassen.

3. Vorrichtung (10) nach einem der vorhergehenden Ansprüche, worin jedes der zumindest eine elektrische und/oder optische Multielektrodenanordnung umfassenden Gitter (1, 4) ferner zumindest einen elektronischen Kreislauf, einen Chip, einen Biosensor, einen optischen Sensor, einen optischen Stimulator, einen elektrischen Sensor oder einen elektrischen Simulator umfasst.

4. Vorrichtung (10) nach einem der vorhergehenden Ansprüche, worin jedes der zumindest eine elektrische und/oder optische Multielektrodenanordnung umfassenden Gitter (1, 4) Elektroden in einer einschichtigen Konfiguration oder alternativ in einer mehrschichtigen Verdrahtungskonfiguration umfasst, so dass dadurch ein miteinander verbundenes Gerüst um das Gitter geformt wird.

5. Vorrichtung (10) nach einem der vorhergehenden Ansprüche, worin das mikrofluidische Perfusionssystem ein Netz von Kanälen aus Polyethylenglykol (PEG) oder Polydimethylsiloxan (PDMS) zur Versorgung des Gewebeschnitts (6) oder der Zellkultur mit Sauerstoff, Wachstumsfaktoren und/oder anderen (bio)chemischen Unterstützung umfasst, um den Gewebeschnitt (6) oder die Zellkultur am Leben zu halten.

6. Vorrichtung (10) nach einem der vorhergehenden Ansprüche, worin das erste Substrat und/oder das zweite Substrat ferner beliebig CMOS-basierte Elektronik, Nanoelektronik, GaN und andere III-IV-basierte Technologie, Post-CMOS-Elektronik und/oder Bioelektronik umfassen.

7. Vorrichtung (10) nach einem der vorhergehenden Ansprüche, worin das erste Substrat und/oder das zweite Substrat in Form eines Gitters Öffnungen (22) in der Größenordnung von 10-50 µm aufweisen.

8. Vorrichtung (10) nach einem der vorhergehenden Ansprüche, worin die Vorrichtung (10) in einer geschlossenen Kammer platziert wird.

9. Vorrichtung (10) nach einem der vorhergehenden Ansprüche, die ferner Zwischensubstrate umfasst, die zwischen dem ersten und zweiten Substrat angeordnet sind und die Form eines Gitters (50) aufweisen, das zumindest eine elektrische und/oder optische Multielektrodenanordnung und optionale Mikrofluidik umfasst.

10. Verwendung einer Vorrichtung (10) nach einem der vorhergehenden Ansprüche zur Stimulierung und/oder Aufzeichnung von Nerven- oder Muskelzellen eines lebenden Gewebes.

11. Verwendung nach Anspruch 10 zur Untersuchung von Aspekten der Muskelphysiologie und/oder von Nervenprozessen.

12. Verfahren zur Herstellung einer gemischten mikrofluidischen
Multielektrodengitteranordnungsvorrichtung (10), wobei das Verfahren Folgendes umfasst:
Erhalt eines ersten und eines zweiten stützenden Substrats,
Bereitstellung einer Gitterkonstruktion in dem ersten und zweiten unterstützenden Substrat,
Bereitstellung einer elektrischen und/oder optischen Multielektrodengitteranordnung auf dem ersten und
zweiten unterstützenden Substrat,
Bereitstellung eines Mittels zur Positionierung und zum Zusammendrücken der ersten und zweiten unterstützenden Substrate und zum Halten eines lebenden Gewebeschnitts oder einer Zellkultur zwischen den beiden Substraten;
und
Bereitstellung eines Gerüsts aus einem Polymermaterial unter dem zweiten Substrat und Bereitstellung eines mikrofluidischen Perfusionssystems in dem Gerüst.

13. Verfahren nach Anspruch 12, das ferner die Bereitstellung von zumindest einem elektrisch mit der Multielektrodengitteranordnung verbundenen Chip, Biosensor, optischen Sensor oder optischen Stimulator umfasst.

14. Verfahren nach einem der Ansprüche 12 oder 13, das ferner das Hinzufügen zumindest eines Zwischensubstrats zwischen den ersten und zweiten unterstützenden Substraten umfasst, wobei das zumindest eine Zwischensubstrat in Form eines Gitter vorliegt, das zumindest eine elektrische und/oder optische Multielektrodenanordnung und optionale Mikrofluidik umfasst.

## Revendications

1. Un dispositif à réseau en grille multi-électrode microfluidique mixte (10) adapté de façon à contenir une coupe tissulaire (6) ou une culture cellulaire et de façon à enregistrer et/ou stimuler des cellules neuronales provenant de ladite coupe tissulaire (6) ou culture cellulaire, le dispositif (10) comprenant :
un premier substrat inférieur sous la forme d'une pile constituée
d'une grille (1) comprenant un réseau (8) multi-électrode électrique et/ou optique et une ossature (2) sous ladite grille (1) comprenant un système de perfusion microfluidique,
un deuxième substrat supérieur sous la forme d'une grille (4)
comprenant au moins un réseau (9) multi-électrode électrique et/ou optique,
un moyen (5) de pression et de positionnement desdits premier et
deuxième substrats de manière conjointe et l'adhésion d'une coupe tissulaire (6) ou d'une culture cellulaire entre lesdits deux substrats.

2. Un dispositif (10) selon la Revendication 1, où le premier substrat et/ou le deuxième substrat contient silicium, verre, silicium sur isolant (SOI), GaN, polymères.

3. Un dispositif (10) selon l'une quelconque des Revendications précédentes, où l'une quelconque desdites grilles (1, 4) comprenant au moins un réseau multi-électrode électrique et/ou optique comprend en outre au moins un élément parmi des circuits électroniques, une puce, un biocapteur, un capteur optique, un stimulateur optique, un capteur électrique, un stimulateur électrique.

4. Un dispositif (10) selon l'une quelconque des Revendications précédentes, où l'une quelconque desdites grilles (1, 4) comprenant au moins un réseau multi-électrode électrique et/ou optique comprenant des électrodes dans une configuration à couche unique ou selon une variante dans une configuration de câblage multicouche, formant ainsi un châssis d'interconnexion autour de la grille.

5. Un dispositif (10) selon l'une quelconque des Revendications précédentes, où le système de perfusion microfluidique comprend un réseau de canaux faits de polyéthylène glycol (PEG) ou de polydiméthylsiloxane (PDMS) pour la fourniture à la coupe tissulaire (6) ou la culture cellulaire d'oxygène, de facteurs de croissance et/ou d'un autre support (bio) chimique de façon à maintenir la coupe tissulaire (6) ou la culture cellulaire en vie.

6. Un dispositif (10) selon l'une quelconque des Revendications précédentes, où le premier substrat et/ou le deuxième substrat comprennent en outre un élément quelconque parmi électronique de type CMOS, nanoélectronique, GaN et autres technologies de type III-IV, électronique post-CMOS et/ou bioélectronique.

7. Un dispositif (10) selon l'une quelconque des Revendications précédentes, où le premier substrat et/ou le deuxième substrat sous la forme d'une grille possède des ouvertures (22) de l'ordre de 10 à 50 µm.

8. Un dispositif (10) selon l'une quelconque des Revendications précédentes, où le dispositif (10) est placé dans une chambre fermée.

9. Un dispositif (10) selon l'une quelconque des Revendications précédentes, comprenant en outre des substrats intermédiaires placés entre ledit premier et ledit deuxième substrat et sous la forme d'une grille (50) comprenant au moins un réseau multi-électrode électrique et/ou optique et en option des micro-fluidiques.

10. L'utilisation d'un dispositif (10) selon l'une quelconque des Revendications précédentes de façon à stimuler et/ou enregistrer des cellules neuronales ou musculaires d'un tissu vivant.

11. Une utilisation selon la Revendication 10 destinée à l'étude d'aspects de physiologie musculaire et/ou de processus neuronaux.

12. Un procédé de fabrication d'un dispositif à réseau en grille multi-électrode microfluidique mixte (10), le procédé comprenant :
l'obtention d'un premier et d'un deuxième substrat porteur,
la fourniture d'une structure de grille dans lesdits premier et
deuxième substrats porteurs,
la fourniture d'un réseau de grille multi-électrode électrique et/ou
optique sur lesdits premier et deuxième substrats porteurs, la fourniture d'un moyen de positionnement et de pression des
premier et deuxième substrats porteurs de manière conjointe et le maintien d'une coupe tissulaire ou d'une culture cellulaire vivante entre les deux substrats, et
la fourniture d'une ossature constituée d'un matériau polymère
sous ledit deuxième substrat et la fourniture d'un système de perfusion microfluidique dans ladite ossature.

13. Un procédé selon la Revendication 12, comprenant en outre la fourniture, de manière électriquement raccordée audit réseau de grille multi-électrode, d'au moins un élément parmi une puce, un biocapteur, un capteur optique, un stimulateur optique.

14. Un procédé selon l'une quelconque des Revendications 12 ou 13, comprenant en outre l'ajout d'au moins un substrat intermédiaire situé entre lesdits premier et deuxième substrats porteurs, le au moins un substrat intermédiaire étant sous la forme d'une grille comprenant au moins un réseau multi-électrode électrique et/ou optique, et des micro-fluidiques en option.
